# EUROPEAN PATENT SPECIFICATION

(11) **EP 4 029 850 B1**
(45) Date of publication and mention of the grant of the patent: **09.08.2023**
(21) Application number: 20862649.9
(22) Date of filing: 08.09.2020
(51) Int. Cl.: C07C 37/68, C07C 37/82, C07C 39/23, C07C 39/19, B01D 15/18, C07C 37/72

(54) **METHOD FOR SIMULTANEOUSLY SEPARATING CANNABIDIVARIN AND CANNABIGEROL**
VERFAHREN ZUR GLEICHZEITIGEN ABTRENNUNG VON CANNABIDIVARIN UND CANNABIGEROL
PROCÉDÉ DE SÉPARATION SIMULTANÉE DE CANNABIDIVARINE ET DE CANNABIGÉROL

(30) Priority: 11.09.2019 CN 201910859985
(43) Date of publication of application: 20.07.2022
(73) Proprietor: Shanghai Tauto Biotech Co., Ltd., Shanghai 201203 (CN)
(72) Inventor: DENG, Li, Shanghai 201203 (CN); YU, Qi, Shanghai 201203 (CN)
(74) Representative: 2K Patentanwälte Blasberg Kewitz & Reichel
(86) International application number: PCT/CN2020/113892
(87) International publication number: WO 2021/047491

(56) References cited:
- WO-A1-2017/214529
- WO-A1-2019/145552
- WO-A1-2019/145552
- CN-A- 107 567 435
- CN-A- 110 590 511
- US-A1- 2018 036 278
- WEISZ ADRIAN ET AL: "Performance comparison of three types of high-speed counter-current chromatographs for the separation of components of hydrophilic and hydrophobic color additives", JOURNAL OF CHROMATOGRAPHY A, vol. 1218, no. 36, 9 September 2011 (2011-09-09), pages 6156-6164, XP028597365, ISSN: 0021-9673, DOI: 10.1016/J.CHROMA.2010.12.034
- Vollner L., D. Bieniek, F. Korte: "HASCHISCH XX. CANNABIDIVARIN, ein neuer Haschisch-Inhaltsstoff", Tetrahedron Letters, no. 3, 1 January 1969 (1969-01-01), pages 145-147, XP055790092,

## Description

### TECHNICAL FIELD

The present invention belongs to the field of cannabinoid processing, and particularly relates to a method for simultaneously separating cannabidivarin and cannabigerol.

### BACKGROUND ART

Cannabidivarin, having the chemical formula of C₁₉H₂₆O₂, is present in industrial hemp. By subjecting the industrial hemp to alcohol extraction and the like, a cannabidivarin-containing extract can be obtained. Cannabidivarin (CBDV) can be used for the treatment of neurological disorders.

Cannabigerol, having the chemical formula of C₂₁H₃₂O₂, is present in industrial hemp. By subjecting the industrial hemp to alcohol extraction and the like, a cannabigerol-containing extract can be obtained. Cannabigerol (CBG) can be used for the treatment of neurological disorders.

Simultaneous separation of cannabidivarin and cannabigerol by high-speed countercurrent chromatography differs from separation of cannabidiol in that: a different solvent system is used to achieve a better separation effect of cannabidivarin and cannabigerol.

High-speed countercurrent chromatography (HSCCC) is a new separation and purification technique based on the principle of liquid-liquid partitioning. It does not require any solid support or carrier. Both the stationary phase and the mobile phase are liquids, without irreversible adsorption.

WO2019/145552 discloses a method for purifying cannabinoids of the CBG or CBD family using counter-current chromatography , either CPC or HSCCC ( high speed counter current chromatgraphy) in which the two-phase system includes hexane :ethanol:water used at ratios of (20:19:1 ) to (20:8:12).

At present, there is no relevant technology for simultaneous separation of cannabidivarin and cannabigerol by high-speed countercurrent chromatography disclosed.

### SUMMARY OF THE INVENTION

The technical problem to be solved by the present invention is to provide a method for simultaneously separating cannabidivarin and cannabigerol. The method uses high-speed countercurrent chromatography for one-step simultaneous separation and purification through a solvent system to obtain cannabidivarin (CBDV) with the purity of greater than 98% and cannabigerol (CBG) with the purity of greater than 97%.

The present invention provides a method for simultaneously separating cannabidivarin and cannabigerol, comprising:
sufficiently oscillating a solvent system, allowing the solvent system to stand, and separately collecting an upper phase and a lower phase; dissolving a commercially available industrial hemp full-spectrum refined oil into the upper phase, performing separation using high-speed countercurrent chromatography with the upper phase as a stationary phase and the lower phase as a mobile phase to obtain a mixture of cannabidivarin and the mobile phase and a mixture of cannabigerol and the mobile phase, respectively, and removing the mobile phase to obtain cannabidivarin and cannabigerol;
wherein the solvent system is obtained by mixing n-hexane or n-heptane, methyl tert-butyl ether, acetonitrile or ethanol, and water in a volume ratio of 5~10: 1∼3: 5∼10: 2∼4.

The separately collected upper phase and lower phase are subjected to ultrasonic degassing treatment.

The conditions for the high-speed countercurrent chromatography are: the rotation direction being forward rotation; the rotation speed of 800 rpm; the column temperature of 25°C; the flow rate of the mobile phase at 5 mL/min; and the detection wavelength of the detector at 214 nm.

The process conditions for removing the mobile phase are: rotary evaporation and vacuum drying at 55°C and -0.085 MPa.

The solvent system of the present invention is determined by the solubility of cannabidivarin and cannabigerol in the immiscible two-phase solvents.

### BENEFICIAL EFFECTS

(1) The present invention uses high-speed countercurrent chromatography to simultaneously separate and purify to obtain cannabidivarin (CBDV) with the purity of greater than 98% and cannabigerol (CBG) with the purity of greater than 97% from the industrial hemp full-spectrum refined oil for the first time.
(2) The high-speed countercurrent chromatography of the present invention has such advantages as no sample loss, no contamination, high efficiency, a large volume of production, and solvent recycling and reuse.
(3) The reagents of the solvent system used in the present invention can all be recycled and reused, and thus are environmentally friendly.

### DETAILED DESCRIPTION OF THE INVENTION

The present invention is further described below in conjunction with specific Examples. It should be appreciated that these Examples are only used to illustrate the present invention rather than to limit the scope of the present invention. In addition, it should be appreciated that, after viewing the content taught by the present invention, a person skilled in the art can make various changes or modifications to the present invention, and these equivalents also fall within the scope defined by the appended claims of the present application.

The reagents and apparatus used in the Examples are as follows:
Reagents: n-hexane, n-heptane, acetonitrile, ethanol, and methyl tert-butyl ether are all analytical reagents manufactured by Sinopharm Chemical Reagent Co., Ltd.; the water is deionized water; and the industrial hemp full-spectrum refined oil is a commercially available product;
Apparatus: the high-speed countercurrent chromatograph is a model TBE-300C high-speed countercurrent chromatograph manufactured by Shanghai Tauto Biotech Co., Ltd.

### Example 1

N-hexane, methyl tert-butyl ether, acetonitrile, and water were mixed in a volume ratio of 6: 3: 6: 3 to prepare a solvent system. The solvent system was added into a separatory funnel to be sufficiently oscillated, and allowed to stand for phase separation, to obtain a two-phase mixture. The upper and lower phases were collected separately, and placed in an ultrasonic oscillator respectively for ultrasonic degassing. The commercially available industrial hemp full-spectrum refined oil was dissolved into the upper phase. High-speed countercurrent chromatography was employed for separation with the upper phase as a stationary phase and the lower phase as a mobile phase, and the chromatographic conditions were set as follows: forward rotation, the rotation speed of 800 rpm; the column temperature of 25°C; the flow rate of the mobile phase at 5 mL/min; and the detection wavelength of the detector at 214 nm. The time when sample introduction was finished was counted as 0 min, a mixture of cannabidivarin (CBDV) and the mobile phase was obtained at 140~200 min, and a mixture of cannabigerol (CBG) and the mobile phase was obtained at 210~280 min. They were placed in a rotary evaporator under the conditions of water bath temperature of 55°C and vacuum pressure of -0.085MPa for rotary evaporation and vacuum drying, and the lower phase was removed, thereby obtaining cannabidivarin (CBDV) and cannabigerol (CBG) products.

The purity of the products obtained in this example was analyzed by high performance liquid chromatography (HPLC), the results showed that: the purity of cannabidivarin (CBDV) was 98.42%, and the purity of cannabigerol (CBG) was 97.53%.

### Example 2

N-heptane, methyl tert-butyl ether, ethanol, and water were mixed in a volume ratio of 5: 3: 5: 4 to prepare a solvent system. The solvent system was added into a separatory funnel to be sufficiently oscillated, and allowed to stand for phase separation, to obtain a two-phase mixture. The upper and lower phases were collected separately, and placed in an ultrasonic oscillator respectively for ultrasonic degassing. The commercially available industrial hemp full-spectrum refined oil was dissolved into the upper phase. High-speed countercurrent chromatography was employed for separation with the upper phase as a stationary phase and the lower phase as a mobile phase, and the chromatographic conditions were set as follows: forward rotation, the rotation speed of 800 rpm; the column temperature of 25°C; the flow rate of the mobile phase at 5 mL/min; and the detection wavelength of the detector at 214 nm. The time when finishing sample introduction was finished was counted as 0 min, a mixture of cannabidivarin (CBDV) and the mobile phase was obtained at 100~140 min, and a mixture of cannabigerol (CBG) and the mobile phase was obtained at 150~200 min. They were placed in a rotary evaporator under the conditions of water bath temperature of 55°C and vacuum pressure of -0.085MPa for rotary evaporation and vacuum drying, and the lower phase was removed, thereby obtaining cannabidivarin (CBDV) and cannabigerol (CBG) products.

The purity of the products obtained in this example was analyzed by high performance liquid chromatography (HPLC), the results showed that: the purity of cannabidivarin (CBDV) was 98.32%, and the purity of cannabigerol (CBG) was 97.15%.

## Claims

1. A method for simultaneously separating cannabidivarin and cannabigerol, comprising:
sufficiently oscillating a solvent system, allowing the solvent system to stand, and separately collecting an upper phase and a lower phase; dissolving a commercially available industrial hemp full-spectrum refined oil into the upper phase, performing separation using high-speed countercurrent chromatography with the upper phase as a stationary phase and the lower phase as a mobile phase to obtain a mixture of cannabidivarin and the mobile phase and a mixture of cannabigerol and the mobile phase, respectively, and removing the mobile phase to obtain cannabidivarin and cannabigerol;
wherein the solvent system is obtained by mixing n-hexane or n-heptane, methyl tert-butyl ether, acetonitrile or ethanol, and water in a volume ratio of 5~10: 1∼3: 5∼10: 2∼4.

2. The method for simultaneously separating cannabidivarin and cannabigerol according to claim 1, wherein the separately collected upper phase and lower phase are subjected to ultrasonic degassing treatment.

3. The method for simultaneously separating cannabidivarin and cannabigerol according to claim 1, wherein the conditions for the high-speed countercurrent chromatography are: the rotation direction being forward rotation; the rotation speed of 800 rpm; the column temperature of 25°C; the flow rate of the mobile phase at 5 mL/min; and the detection wavelength of the detector at 214 nm.

4. The method for simultaneously separating cannabidivarin and cannabigerol according to claim 1, wherein the process conditions for removing the mobile phase are: rotary evaporation and vacuum drying at 55°C and -0.085 MPa.

## Patentansprüche

1. Verfahren zur gleichzeitigen Trennung von Cannabidivarin und Cannabigerol, umfassend:
ausreichendes Schütteln eines Lösungsmittelsystems, dann Stehenlassen des Lösungsmittelsystems und separates Sammeln einer oberen Phase und einer unteren Phase; Auflösen eines kommerziell erhältlichen raffinierten Vollspektrum-Industriehanföls in der oberen Phase, und Durchführen der Trennung unter Verwendung von Hochgeschwindigkeits-Gegenstromchromatographie, wobei die obere Phase als stationäre Phase und die untere Phase als mobile Phase dient, um eine Mischung aus Cannabidivarin und der mobilen Phase zu erhalten bzw. eine Mischung aus Cannabigerol bzw. der mobilen Phase zu erhalten, und Entfernen der mobilen Phase, um Cannabidivarin und Cannabigerol zu erhalten;
wobei das Lösungsmittelsystem durch Vermischen von n-Hexan oder n-Heptan, Methyl-tert-butylether, Acetonitril oder Ethanol und Wasser in einem Volumenverhältnis von 5-6:1-3:5-6:3-4 erhalten wird.

2. Verfahren zur gleichzeitigen Trennung von Cannabidivarin und Cannabigerol nach Anspruch 1, wobei die separate gesammelte obere Phase und untere Phase einer Ultraschall-Entgasungsbehandlung ausgesetzt werden.

3. Verfahren zur gleichzeitigen Trennung von Cannabidivarin und Cannabigerol nach Anspruch 1, wobei die Bedingungen für die Hochgeschwindigkeits-Gegenstromchromatographie sind: die Rotationsrichtung ist Vorwärtsrotation; die Rotationsgeschwindigkeit beträgt 800 U/min; die Säulentemperatur beträgt 25°C; die Flussrate der mobilen Phase ist 5 ml/min; und die Nachweiswellenlänge des Detektors ist 214 nm.

4. Verfahren zur gleichzeitigen Trennung von Cannabidivarin und Cannabigerol nach Anspruch 1, wobei die Prozessbedingungen für die Entfernung der mobilen Phase sind: Durchführen von Rotationsverdampfung und Vakuumtrocknung bei 55°C und -0,085 MPa.

## Revendications

1. Procédé pour séparer simultanément la cannabidivarine et le cannabigérol, comprenant :
osciller suffisamment un système de solvants, laisser reposer le système de solvants et collecter séparément une phase supérieure et une phase inférieure ; dissoudre une huile raffinée à spectre complet de chanvre industriel disponible commercialement dans la phase supérieure, effectuer une séparation par chromatographie à contre-courant à grande vitesse avec la phase supérieure comme phase statique et la phase inférieure comme phase mobile pour obtenir un mélange de la cannabidivarine et de la phase mobile et un mélange du cannabigérol et de la phase mobile respectivement, et éliminer la phase mobile pour obtenir la cannabidivarine et le cannabigérol ;
dans lequel le système de solvants est obtenu en mélangeant le n-hexane ou le n-heptane, le méthyl tert-butyl éther, l'acétonitrile ou l'éthanol, et l'eau dans un rapport en volume de 5 - 6 : 1- 3 : 5 - 6 : 3 - 4.

2. Procédé pour séparer simultanément la cannabidivarine et le cannabigérol selon la revendication 1, dans lequel la phase supérieure et la phase inférieure collectées séparément sont soumises à un traitement de dégazage à ultrasons.

3. Procédé pour séparer simultanément la cannabidivarine et le cannabigérol selon la revendication 1, dans lequel les conditions pour la chromatographie à contre-courant à grande vitesse sont : le sens de rotation étant le sens de rotation vers l'avant ; la vitesse de rotation de 800 tr/min ; la température de la colonne de 25 °C ; le débit de la phase mobile de 5 mL/min ; et la longueur d'onde de détection du détecteur de 214 nm.

4. Procédé pour séparer simultanément la cannabidivarine et le cannabigérol selon la revendication 1, dans lequel les conditions de processus d'élimination de la phase mobile sont : une évaporation rotative et un séchage sous vide à 55 °C et à -0,085 MPa.
